# EUROPÄISCHE PATENTSCHRIFT

(11) **EP 0 928 192 B1**
(45) Veröffentlichungstag und Bekanntmachung des Hinweises auf die Patenterteilung: **02.05.2002**
(21) Anmeldenummer: 97909278.0
(22) Anmeldetag: 16.09.1997
(51) Int. Cl.: A61K 31/44, A61K 9/00

(54) **ANTIMYKOTISCHES GEL MIT HOHER WIRKSTOFFFREISETZUNG**
ANTIMYCOTIC GEL WITH HIGH ACTIVE SUBSTANCE RELEASE
GEL ANTIMYCOTIQUE A FORTE LIBERATION DE PRINCIPE ACTIF

(30) Priorität: 27.09.1996 DE 19639816
(43) Veröffentlichungstag der Anmeldung: 14.07.1999
(73) Patentinhaber: Aventis Pharma Deutschland GmbH, 65929 Frankfurt am Main (DE)
(72) Erfinder: BOHN, Manfred, D-65719 Hofheim (DE); KRAEMER, Karl, Theodor, D-63225 Langen (DE); MARKUS, Astrid, D-65835 Liederbach (DE)
(86) Internationale Anmeldenummer: EP9705068
(87) Internationale Veröffentlichungsnummer: WO9813042

(56) Entgegenhaltungen:
- EP-A- 0 218 410
- EP-A- 0 313 305
- EP-A- 0 680 745
- WO-A-95/17165
- WO-A-96/29056
- DE-A- 3 140 954
- DE-A- 3 826 914
- GB-A- 2 208 149
- DATABASE WPI Week 9542 Derwent Publications Ltd., London, GB; AN 95-325488 XP002052245 & JP 07 223 971 A (POLA CHEM IND. INC.) , 22.August 1995

## Beschreibung

Die vorliegende Erfindung betrifft ein topisch applizierbares antimykotisch wirksames Präparat mit hoher Wirkstofffreisetzung in Form einer Gelzubereitung, welche mindestens eine antimykotisch wirksame Substanz aus der Klasse der Hydroxypyridone und mindestens einem hydrophilen Gelbildner enthält.

In der Patentanmeldung WO 95/17165 werden pharmazeutische Formulierungen beschrieben, die eine antimikrobiell wirkende Verbindung, eine befeuchtende Komponente und einen hydrophilen Gelbildner enthalten. In der Europäischen Patentanmeldung 0680745 werden dermatologische und kosmetische Formulierungen offenbart, die zur Verringerung des Haarausfalls dienen, die eine antimykotisch wirksame Verbindung und einen halogenierten antibakteriell wirksamen Stoff enthalten. Die Patentanmeldung GB 2 208 149 beschreibt ein Kombinationspräparat, enthaltend ein Pyridonderivat und ein Markrolidantibiotika.

Für die topische Behandlung von Mykosen, vor allem Mykosen der Haut, sind bereits verschiedene Zubereitungsformen von Hydroxypyridonderivaten wie Lösungen, Salben und Puder bekannt. Eine optimale Behandlung von Hautmykosen ist jedoch mit den bislang bekannten Zubereitungsformen von Hydroxypyridonen aus den verschiedensten Gründen nicht uneingeschränkt möglich.

Topisch applizierbare flüssige Zubereitungen umfassen im allgemeinen klare wäßrige oder wäßrig-alkoholische Lösungen. Sie werden entweder auf die Hautoberfläche aufgepinselt oder für Waschungen oder Bäder verwendet. Insbesondere finden sie Anwendung bei jenen Hautregionen, die von dichtem Haarwuchs bedeckt sind, da für diese Gebiete Salben oder Puder nicht geeignet sind. Darüber hinaus gelangen sie bei solchen Hautarealen zur Anwendung, für die andere Arzneiformen aus kosmetischen Gründen nicht gerne verwendet werden, z. B. im Gesicht oder an stark bewegten Körperstellen (z. B. Ellbogen, Knie u.s.w.).

Die Freisetzungsrate des Wirkstoffes aus Lösungen ist im allgemeinen hoch, da nach Applikation durch Abdunsten der Vehikelbestandteile ein starkes Konzentrationsgefälle zwischen Zubereitung und Haut entsteht, was letztendlich zu einer hohen Wirkstoffaufnahme durch die Haut und damit zu einer hohen Wirksamkeit führt.

Im Hinblick auf ihre Anwendungseigenschaften sind Lösungen dagegen eher als weniger günstig zu bewerten, da sie aufgrund ihres flüssigen Aggregatzustandes insbesondere im Gesicht nur schwer zu handhaben sind und ein gezieltes Auftragen auf begrenzte Hautareale nicht möglich ist.

Salben oder halbfeste Arzneimittelformen sind Darreichungsformen, die im allgemeinen im Temperaturbereich zwischen Raumtemperatur und Hauttemperatur streichfähig sind und dadurch von den flüssigen Darreichungsformen und denen mit Feststoffcharakter differenziert werden können. Basierend auf den Stoffeigenschaften der Hauptvehikelsubstanzen werden unter Salben im allgemeinen wasserfreie Fettgrundlagen oder aus einer öligen und wäßrigen Phase bestehende Emulsionen, die durch einen Emulgator stabilisiert sind, verstanden.

Aufgrund ihrer halbfesten Konsistenz lassen sich Salbenzubereitungen - im Gegensatz zu Lösungen - sehr gezielt auf begrenzte Hautareale auftragen. Bedingt durch den Gehalt an Fettbestandteilen ist jedoch die Freisetzung der lipophilen Hydroxypyridonderivate aus den Salbenbestandteilen stark eingeschränkt. Der Behandlungserfolg nach Salbenapplikation wird weiterhin dadurch beeinträchtigt, daß Salben gewöhnlich auf der Haut keinen wischfesten Film hinterlassen. Das aufgetragene Produkt kann somit bei Berührung mit der Kleidung oder Bettwäsche leicht wieder entfernt werden und steht damit für eine erfolgreiche Therapie nicht mehr zur Verfügung.

Puderzubereitungen dienen in erster Linie der Adsorption vermehrter Sekretion und der Trockenhaltung der Haut; ein Gesichtspunkt, der insbesondere bei der Behandlung von Hautmykosen eine bedeutende Rolle spielt. Die Anwendung von Puderzubereitungen ist aus praktischen Gründen fast ausschließlich auf die Behandlung von Fußmykosen beschränkt.

Es wurde nun gefunden, daß Gelformulierungen von Hydroxypyridonderivaten, die Lösungsmittel und hydrophile Gelbildner sowie übliche Formulierungshilfsstoffe enthalten, eine hohe Freisetzung des Wirkstoffes und damit eine verbesserte Wirkung durch das Erreichen von hohen Konzentrationen des Wirkstoffes in der Haut ermöglichen. Die Zubereitungen lassen sich ferner aufgrund ihrer halbfesten Konsistenz leicht und gezielt auf die betroffenen Hautareale auftragen und zeigen darüber hinaus den besonders bei der Behandlung von Fußmykosen gewünschten Austrocknungseffekt.

Die Erfindung betrifft daher die Verwendung der pharmazeutischen Zubereitung, enthaltend einen hydrophilen Gelbildner, Wasser, ein Netzmittel und ein Spreitmittel und eine Verbindung der Formel I 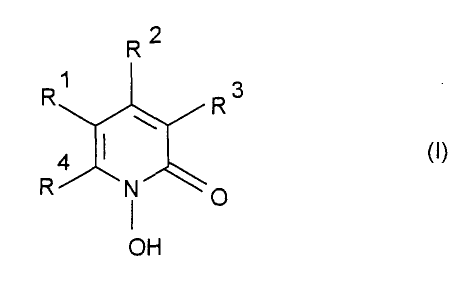 oder ein physiologisch verträgliches Salz der Verbindung der Formel I,
wobei R¹, R² und R³, die gleich oder verschieden sind, Wasserstoffatom oder Alkyl mit 1 bis 4 Kohlenstoffatomen bedeuten, und R⁴ einen gesättigten Kohlenwasserstoffrest mit 6 bis 9 Kohlenstoffatomen bedeutet,
zur Herstellung eines Arzneimittels zur Behandlung und Prophylaxe von Hautmykosen.

Bevorzugt ist die Verwendung einer pharmazeutischer Zubereitung, wobei R⁴ für einen gesättigten Kohlenwasserstoff mit 6 bis 9 Kohlenstoffatomen steht, einer der Reste R¹ und R³ Wasserstoffatom und der andere Wasserstoffatom, Methyl oder Ethyl bedeutet und R² Alkylrest mit 1 oder 2 Kohlenstoffatomen bedeutet.

Insbesondere bevorzugt ist die Verwendung einer pharmazeutischen Zubereitung, die dadurch gekennzeichnet, daß die Verbindung der Formel I in der Position R⁴ einen cyclischen Rest enthält.

Ferner ist die Verwendung einer pharmazeutischen Zubereitung bevorzugt, die dadurch gekennzeichnet, daß R⁴ ein Cyclohexylrest oder -CH₂-CH(CH₃)-CH₂-(C(CH₃)₃ ist.

Der Begriff "gesättigt" bezeichnet hierbei solche Reste, die keine aliphatischen Mehrfachbindungen, also keine ethylenischen oder actetylenischen Bindungen enthalten.

Als geeignete Verbindungen der Formel I seien beispielsweise genannt 1-Hydroxy-4-methyl-6-n-hexyl-, -6-iso-hexyl-, -6-n-heptyl- oder -6-iso-heptyl-2-pyridon, 1-Hydroxy-4-methyl-6-octyl- oder -6-iso-octyl-2-pyridon, insbesondere als 1-Hydroxy-4-methyl-6-(2,4,4-trimethylpentyl)-2-pyridon, 1-Hydroxy-4-methyl-6-cyclohexyl-2-pyridon, 1-Hydroxy-4-methyl-6-cyclohexylmethyl- oder -6-cyclohexylethyl-2-pyridon, wobei der Cyclohexylrest jeweils auch einen Methylrest tragen kann, 1-Hydroxy-4-methyl-6-(2-bicyclo[2,2,1]heptyl)-2-pyridon, 1-Hydroxy-3,4-dimethyl-6-benzyloder-6-dimethylbenzyl-2-pyridon und 1-Hydroxy-4-methyl-6-(ß-phenyl-ethyl)-2-pyridon.

Mit dem erfindungsgemäßen verwendeten Arzneimittel läßt sich bei den Behandlungen von Hautmykosen eine durchgreifende Heilung erzielen. Das erfindungsgemäße verwendete Arzneimittel eignet sich auch zur prophylaktischen Anwendung gegen Hautmykosen.

Der Gehalt an der Verbindung der Formel I in der erfindungsgemäßen pharmazeutischen Zubereitung ist von der Struktur einer jeden Verbindung der Formel I und damit von deren Freigabe aus dem Gel, seinem Penetrationsverhalten in der Haut und seinen antimikrobiellen Eigenschaften abhängig.

In der erfindungsgemäßen pharmazeutischen Zubereitung ist die Verbindung der Formel I im allgemeinen in einer Menge von 0,05 bis 2 Gewichtsprozent, vorzugsweise 0,1 bis 1 Gew.-%, enthalten.

Als Gelbildner kommen native Substanzen wie Gelatine, Pektin, Karrageen, Agar, Tragant und Alginate, halbsynthetische Gelbildner wie Celluloseether (Methylcellulose, Ethylcellulose, Hydroxyethylcellulose, Hydroxypropylcellulose, Natriumcarboxymethylcellulose), Stärkederivate und Pektinderivate sowie vollsynthetische Gelbildner wie Polyacrylate, Polymethacrylate, Polyvinylalkohol und Polyvinylpyrrolidone in Frage. Besonders geeignet sind Polyacrylate. Sie werden in Mengen von 0,3 bis 2,0 Gewichtsteilen auf 100 Gewichtsteile Endprodukt eingesetzt.

Als Lösungsmittel sind Wasser und auch alle mit Wasser mischbaren Lösungsmittel geeignet. In Betracht kommen z. B. Alkanole wie Ethanol oder Isopropylalkohol, sowie Propylenglykol und Dimethylsulfoxid. Es können bei der Herstellung der erfindungsgemäßen Formulierungen ein oder mehrere Lösungsmittel eingesetzt werden.

Als zusätzlichen Lösungsvermittler eignen sich für die erfindungsgemäße pharmazeutische Zubereitung:
Benzylalkohole, 2-Octyldodecanol, Adipate, Propylenglykol und Glycerin.
Diese Lösungvermittler sind in den erfindungsgemäßen Zubereitungen von 1 bis 15 Gewichtsprozent (Gew.-%) enthalten.

Als weitere Hilfsmittel sind Emulgatoren, Netz- und Spreitmittel geeignet.

Die Herstellung der Zubereitungen erfolgt in an sich bekannter Weise durch Zusammengeben der einzelnen Komponenten und einer - soweit erforderlich - der jeweiligen Zubereitung angepaßten Weiterverarbeitung.

Die vorliegende Erfindung wird durch die folgenden Beispiele näher erläutert, jedoch nicht auf diese beschränkt. Soweit nichts anderes vermerkt, sind die Mengenangaben auf das Gewicht bezogen.

### Beispiel 1

Eine erfindungsgemäße Zubereitung weist folgende Zusammensetzung auf:

| | |
|---|---|
| 1-Hydroxy-4-methyl-6-(2,4,4-trimethyipentyl)-2(1H)pyridon | 0,50 % |
| Hydroxyethylcellulose | 1,50 % |
| Polyethylenglykol-7 Glycerylcocoat | 5,00 % |
| 1,2-Propylenglykol | 10,00 % |
| Isopropylalkohol | 20,00 % |
| Demineralisiertes Wasser | 63.00 % |

### Beispiel 2

Eine erfindungsgemäße Zubereitung weist folgende Zusammensetzung auf:

| | |
|---|---|
| 1-Hydroxy-4-methyl-6-cyclohexyl-2(1H)pyridon | 1,00 % |
| Polyacrylsäurepolymer (z. B. Carbomer 934 P) | 0,70 % |
| Natriumhydroxid | 0,20 % |
| Natriumdioctylsulfosuccinat | 0,05 % |
| 2-Octyldodecanol | 7,50 % |
| Isopropylalkohol | 25,00 % |
| Demineralisiertes Wasser | 65,55 % |

### Beispiel 3

Eine erfindungsgemäße Zubereitung weist folgende Zusammensetzung auf:

| | |
|---|---|
| 1-Hydroxy-4-methyl-6-cyclohexyl-2(1H)pyridon | 0,50 % |
| Polyacrylsäurepolymer (z. B. Carbomer 940) | 0,50 % |
| Natriumhydroxid | 0,20 % |
| Polyoxyethylen(20)sorbitanmonostearat | 3,50 % |
| Isopropylmyristat | 10,00 % |
| Ethanol | 20,00 % |
| Demineralisiertes Wasser | 65,30 % |

### Beispiel 4

Eine Salbenzubereitung aus dem Stand der Technik weist folgende Zusammensetzung auf:

| | |
|---|---|
| 1-Hydroxy-4-methyl-6-cyclohexyl-2(1H)pyridon | 1,00 % |
| Vaseline | 20,00 % |
| Stearylalkohol | 15,00 % |
| 2-Octyldodecanol | 10,00 % |
| Polyoxyethylen(20)sorbitanmonostearat | 3,50 % |
| Sorbitanmonostearat | 1,50 % |
| Demineralisiertes Wasser | 49,00 % |

### Beispiel 6

### Wirksamkeitsprüfung

Testung der Wirkstofffreisetzung der erfindungsgemäßen pharmazeutischen Zubereitung im Penetrationsmodell mit exzidierter Schweinehaut.

Die Testung der Wirkstofffeisetzung aus den erfindungsgemäßen Mitteln erfolgte im Penetrationsmodell an exzidierter Schweinehaut. Dabei wird indirekt über die Bestimmung der Penetrationstiefe mittels einer mikrobiologischen Bestimmungsmethode auf die Wirkstofffreisetzung aus den erfindungsgemäßen Mitteln geschlossen:

Von Schlachtschweinen wurden vor der Brühung der getöteten Tiere größere Flächen von Rückenhaut exzidiert, mit feuchtem Papier und Plastikfolie umwickelt und bei -20°C bis zum Versuch tiefgefroren.

Die Hautoberfläche wurde vor dem Versuch von Fettgewebe befreit, rasiert und für 60 Minuten zu Desinfektionszwecken mit Isopropanol behandelt. Für jeden Versuchsansatz wurde ein gesondertes Hautstück (ca. 2 x 3 cm) verwendet. Die Hautoberfläche wurde mit verschiedenen Verbindungen der Formel I enthaltenen Zubereitungen behandelt. Nach Ende der verschiedenen Einwirkzeiten (0,5, 1 und 4 Stunden) wurden die Produkte durch Waschen von der Hautoberfläche entfernt. Um das unterschiedliche Penetrationsvermögen der Wirkstoffe - bzw. das unterschiedliche Freisetzungsvermögen der Zubereitungen - zu untersuchen, wurden auf jeweils 3 nebeneinanderliegenden Bahnen die Hautstücke mit Tesafilm 2 x, 6 x und 10 x abgestrippt. Jede Bahn wurde anschließend 10 x punktförmig mit einer Suspension aus Trichophyton mentagrophytes 100/25 (ca. 200 Mikrokonidien pro Impfpunkt) inokuliert. Anschließend wurden die Hautstücke auf Wasseragar mit Penicillin-, Streptomycin- und Cycloheximidzusatz 7 Tage bei 28°C bebrütet. Vom 4. Inkubationstag an wurde täglich makroskopisch abgelesen.

### Ergebnis:

Nach einer Einwirkungszeit der wirkstoffhattigen Gelzubereitungen, gemäß der Beispiele 1 bis 4, von 4 Stunden sind die Hautstücke auf allen Abschnitten - im Gegensatz zu den entsprechenden Placebozubereitungen - makroskopisch pilzfrei.

Die Einwirkungszeit von 4 Stunden ist für die nicht erfindungsgemäße wirkstoffhaltige Salbenzubereitung, nach Beispiel 5, die gemäß dem Stand der Technik hergestellt wurde, nicht ausreichend, um die Makrokonidien auf den inokulierten Segmenten abzutöten.

## Patentansprüche

1. Verwendung der pharmazeutischen Zubereitung, enthaltend einen hydrophilen Gelbildner, Wasser, ein Netzmittel und ein Spreitmittel und eine Verbindung der Formel I 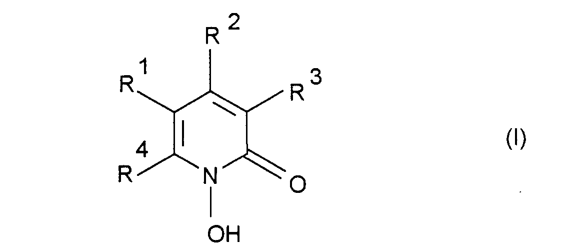 oder ein physiologisch verträgliches Salz der Verbindung der Formel I,
wobei R¹, R² und R³, die gleich oder verschieden sind, Wasserstoffatom oder Alkyl mit 1 bis 4 Kohlenstoffatomen bedeuten, und R⁴ einen gesättigten Kohlenwasserstoffrest mit 6 bis 9 Kohlenstoffatomen bedeutet, zur Herstellung eines Arzneimittels zur Behandlung und Prophylaxe von Hautmykosen.

2. Verwendung gemäß Anspruch 1, **dadurch gekennzeichnet, daß** R⁴ für einen gesättigten Kohlenwasserstoff mit 6 bis 9 Kohlenstoffatomen steht, einer der Reste R¹ und R³ Wasserstoffatom und der andere Wasserstoffatom, Methyl oder Ethyl bedeutet und
R² Alkylrest mit 1 oder 2 Kohlenstoffatomen bedeutet.

3. Verwendung gemäß Anspruch 1 oder 2, **dadurch gekennzeichnet, daß** die Verbindung der Formel I in der Position R⁴ einen cyclischen Rest enthält.

4. Verwendung gemäß Anspruch 3, **dadurch gekennzeichnet, daß** R⁴ ein Cyclohexylrest oder -CH₂-CH(CH₃)-CH₂-C(CH₃)₃ ist.

5. Verwendung gemäß einem oder mehreren der Ansprüche 1 bis 4, **dadurch gekennzeichnet, daß** als hydrophiler Gelbildner native Substanzen wie Gelatine, Pektin, Tragant, Agar, Karrageen oder Alginat, halbsynthetische Verbindungen wie Celluloseether, z.B. Methylcellulose, Ethylcellulose, Hydroxyethylcellulose, Hydroxypropylcellulose oder Natriumcarboxymethylcellulose, Stärkederivate oder Pektinderivate sowie vollsynthetische Gelbildner wie Polyacrylate, Polymethacrylate, Polyvinylalkohol oder Polyvinylpyrrolidone oder Mischungen der hydrophilen Gelbildner eingesetzt werden.

6. Verwendung gemäß Anspruch 5, **dadurch gekennzeichnet, daß** Polyacrylat als hydrophiler Gelbildner eingesetzt wird.

7. Verwendung gemäß einem oder mehreren der Ansprüche 1 bis 6, **dadurch gekennzeichnet, daß** zusätzlich Lösungsvermittler aus der Gruppe Benzylalkohol, 2-Octyldodecanol, Propylenglycol, Adipate und Glycerin eingesetzt werden.

8. Verwendung gemäß einem oder mehreren der Ansprüche 1 bis 7, **dadurch gekennzeichnet, daß** zusätzlich ein mit Wasser vermischbares Lösungsmittel wie Alkanole, z.B. Ethanol und/oder Isopropylalkohol, sowie Propylenglykol oder Dimethylsulfoxid eingesetzt wird.

9. Verwendung gemäß einem oder mehreren der Ansprüche 1 bis 8, **dadurch gekennzeichnet, daß** die Verbindung der Formel I in einer Menge von 0,05 bis 2 Gewichtsprozent, vorzugsweise von 0,1 bis 1 Gewichtsprozent und der hydrophile Gelbildner in einer Menge von 0,3 bis 2 Gewichtsprozent enthalten ist

10. Verwendung gemäß Anspruch 1, **dadurch gekennzeichnet, daß** zusätzlich ein Emulgator eingesetzt wird.

11. Verwendung gemäß Anspruch10, **dadurch gekennzeichnet, daß** als Emulgator Polyoxyethylen(20)sorbitanmonostearat eingesetzt wird

12. Verwendung gemäß Anspruch1, **dadurch gekennzeichnet, daß** als Spreitmittel Polyethylenglykol-7-Glycerococoat, 2-Octyldodecanol oder Isopropylmyristat eingesetzt wird.

13. Verwendung gemäß Anspruch 1, **dadurch gekennzeichnet, daß** als Netzmittel Natriumdioctylsulfosuccinat eingesetzt wird.

## Claims

1. The use of the pharmaceutical preparation comprising a hydrophilic gel-forming agent, water, a wetting agent and a spreading agent and a compound of the formula I 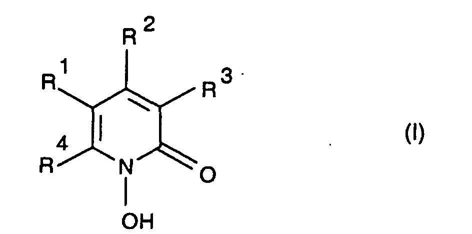 or a physiologically tolerable salt of the compound of the formula I,
where R¹, R² and R³, which are identical or different, are a hydrogen atom or alkyl having 1 to 4 carbon atoms, and R⁴ is a saturated hydrocarbon radical having 6 to 9 carbon atoms, for preparing a pharmaceutical for the treatment and prophylaxis of dermatomycoses.

2. The use as claimed in claim 1, wherein R⁴ is a saturated hydrocarbon having 6 to 9 carbon atoms, one of the radicals R¹ and R³ is a hydrogen atom and the other is a hydrogen atom, methyl or ethyl and
R² is an alkyl radical having 1 or 2 carbon atoms.

3. The use as claimed in claim 1 or 2, wherein. the compound of the formula I contains a cyclic radical in the position R⁴.

4. The use as claimed in claim 3, wherein R⁴ is a cyclohexyl radical or -CH₂-CH(CH₃)-CH₂-C(CH₃)₃.

5. The use as claimed in one or more of claims 1 to 4, wherein the hydrophilic gel-forming agents employed are native substances such as gelatin, pectin, tragacanth, agar, carrageenan or alginate, semisynthetic compounds such as cellulose ethers, e.g. methylcellulose, ethylcellulose, hydroxyethylcellulose, hydroxypropylcellulose or sodium carboxymethylcellulose, starch derivatives or pectin derivatives and also fully synthetic gel-forming agents such as polyacrylates, polymethacrylates, polyvinyl alcohol or polyvinylpyrrolidones or mixtures of the hydrophilic gel-forming agents.

6. The use as claimed in claim 5, wherein polyacrylate is employed as the hydrophilic gel-forming agent.

7. The use as claimed in one or more of claims 1 to 6, wherein solubilizers from the group consisting of benzyl alcohol, 2-octyldodecanol, propylene glycol, adipates and glycerol are additionally employed.

8. The use as claimed in one or more of claims 1 to 7, wherein a water-miscible solvent such as an alkanol, e.g. ethanol and/or isopropyl alcohol and also propylene glycol or dimethyl sulfoxide is additionally employed.

9. The use as claimed in one or more of claims 1 to 8, wherein the compound of the formula I is contained in an amount from 0.05 to 2% by weight, preferably from 0.1 to 1% by weight, and the hydrophilic gel-forming agent is contained in an amount from 0.3 to 2% by weight.

10. The use as claimed in claim 1, wherein an emulsifier is additionally employed.

11. The use as claimed in claim 10, wherein the emulsifier employed is polyoxyethylene(20) sorbitan monostearate.

12. The use as claimed in claim 1, wherein the spreading agent employed is polyethylene glycol-7 glycerylcocoate, 2-octyldodecanol or isopropyl myristate.

13. The use as claimed in claim 1, wherein the wetting agent employed is sodium dioctylsulfosuccinate.

## Revendications

1. Utilisation de la préparation pharmaceutique contenant un agent gélifiant hydrophile, de l'eau, un agent mouillant et un agent dispersant et un composé de la formule I 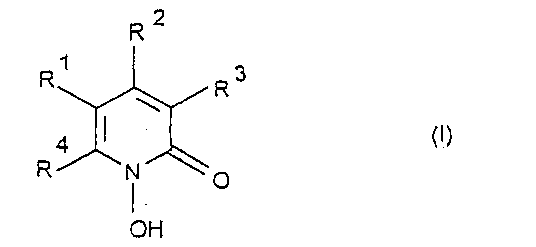 ou un sel physiologiquement acceptable du composé de la formule I,
dans laquelle R¹, R² et R³, qui sont identiques ou différents, représentent un atome d'hydrogène ou un groupe alkyle avec 1 à 4 atomes de carbone, et R⁴ représente un résidu hydrocarboné saturé avec 6 à 9 atomes de carbone,
pour la préparation d'un médicament pour le traitement et la prophylaxie de mycoses de la peau.

2. Utilisation selon la revendication 1, **caractérisée en ce que** R⁴ représente un matériau hydrocarboné saturé avec 6 à 9 atomes de carbone, un des groupes R¹ et R³ représente un atome d'hydrogène et l'autre un atome d'hydrogène, un groupe méthyle ou éthyle et
R² représente un groupe alkyle avec 1 ou 2 atomes de carbone.

3. Utilisation selon la revendication 1 ou 2, **caractérisée en ce que** le composé de la formule I contient dans la position R⁴ un groupe cyclique.

4. Utilisation selon la revendication 3, **caractérisée en ce que** R⁴ est un groupe cyclohexyle ou -CH₂-CH(CH₃)-CH₂-C(CH₃)₃.

5. Utilisation selon une ou plusieurs des revendications 1 à 4, **caractérisée en ce qu'**on utilise comme agent gélifiant hydrophile des substances natives comme la gélatine, la pectine, la gomme adragante, l'agar-agar, le carraghénate ou l'alginate, des composés semisynthétiques comme les éthers de cellulose, par exemple la méthylcellulose, l'éthylcellulose, l'hydroxyéthylcellulose, l'hydroxypropylcellulose ou la carboxyméthylcellulose de sodium, des dérivés de l'amidon, ou des dérivés de la pectine ainsi que des agents gélifiants entièrement synthétiques comme les polyacrylates, les poly-méthacrylates, l'alcool polyvinylique ou la polyvinyl-pyrrolidone ou des mélanges des agents gélifiants hydrophiles.

6. Utilisation selon la revendication 5, **caractérisée en ce qu'**on utilise du polyacrylate comme agent gélifiant hydrophile.

7. Utilisation selon une ou plusieurs des revendications 1 à 6, **caractérisée en ce qu'**on utilise en plus un agent de solubilisation choisi dans le groupe de l'alcool benzylique, du 2-octyldodécanol, du propylèneglycol, des adipates et de la glycérine.

8. Utilisation selon une ou plusieurs des revendications 1 à 7, **caractérisée en ce qu'**on utilise en plus un solvant miscible à l'eau comme les alcanols, par exemple l'éthanol et/ou l'alcool isopropylique, ainsi que le propylèneglycol ou le diméthylsulfoxyde.

9. Utilisation selon une ou plusieurs des revendications 1 à 8, **caractérisée en ce que** le composé de la formule I est contenu en une quantité variant de 0,05 à 2 pour cent en poids, de préférence de 0,1 à 1 pour cent en poids et l'agent gélifiant hydrophile en une quantité variant de 0,3 à 2 pour cent en poids.

10. Utilisation selon la revendication 1, **caractérisée en ce qu'**on utilise en plus un agent émulsionnant.

11. Utilisation selon la revendication 10, **caractérisée en ce qu'**on utilise comme agent émulsionnant le monostéarate de polyoxyéthylène(20) sorbitane.

12. Utilisation selon la revendication 1, **caractérisée en ce qu'**on utilise comme agent dispersant le 7-glycérococoate de polyéthylèneglycol-7, le 2-octyldodécanol ou le myristate d'isopropyle.

13. Utilisation selon la revendication 1, **caractérisée en ce qu'**on utilise comme agent mouillant le sulfosuccinate de dioctyle de sodium.
